# EUROPEAN PATENT APPLICATION

(11) **EP 4 813 426 A1**
(43) Date of publication of application: **30.09.2026**
(21) Application number: 25425014.5
(22) Date of filing: 28.03.2025
(51) Int. Cl.: A61M 15/00, A61M 15/06

(54) **VAPORIZATION DEVICE FOR USE IN THE ADMINISTRATION BY INHALATION OF A LIQUID COMPRISING AT LEAST ONE ACTIVE INGREDIENT**

(71) Applicant: Gervasoni, Cristina, 25032 Chiari (BS) (IT)
(72) Inventor: Gervasoni, Cristina, 25032 Chiari (BS) (IT)
(74) Representative: Botti & Ferrari S.p.A.

(57) **Abstract**

A device (1;20) for the vaporization and the administration by inhalation of a liquid is described, the device comprising:
- a tank (3) containing a liquid to be vaporized, said liquid comprising at least one therapeutic, nutraceutical, food-supplement, phytotherapeutic, aromatherapeutic, cosmetic or general-wellness active ingredient or said liquid being a liquid classified as a medical device,
- a vaporization unit comprising a heating element (4) or an ultrasonic transducer to vaporize said liquid in fluid communication with said tank (3), said heating element being selected from a coil atomizer, a ceramic atomizer and a mesh atomizer;
- a mouthpiece (7) connected to an end of said vaporization unit and in fluid communication with said heating element (4) or said ultrasonic transducer,
- power-supply means in electrical communication with said heating element (4) or said ultrasonic transducer,
- at least one opening (10) in fluid communication with said heating element (4) or said ultrasonic transducer to send an air flow from the outside to said heating element (4) or said ultrasonic transducer,
- a command-and-control unit in electrical communication with said power-supply means and configured to control the temperature of said heating element (4) or the power of said ultrasonic transducer.

## Description

### Field of application

The present invention refers to a vaporization device suitable to be used in the administration by inhalation of a liquid or a liquid formulation comprising at least one therapeutic, nutraceutical, food-supplement, phytotherapeutic, aromatherapeutic, cosmetic or general-wellness active ingredient or for the use in the administration by inhalation of a liquid classified as a medical device.

The device according to the invention uses a vaporization technology typical of devices of the vape type commonly used to vaporize liquids containing nicotine and allows to obtain controlled vaporization of liquids containing active ingredients as indicated above, to improve the bioavailability and optimize the administration thereof.

### Prior art

It is known that there are many devices using different technologies for vaporization and administration of substances by inhalation.

In this respect, vape-type devices widely used to vaporize nicotine-containing liquids are known. In those devices, the heating element is usually a coil, ceramic or mesh atomizer which allows the vaporization of the liquid by direct heating. Although said devices are very widespread, their application is essentially limited to the consumption of nicotine or aromatic substances.

Moreover, medical inhalers are also known, which are devices used primarily to administer respiratory drugs, such as bronchodilators for asthma and COPD (Chronic Obstructive Pulmonary Disease") treatment. Those devices use primarily techniques of low-temperature nebulization or vaporization, but their use is limited to the administration of drugs for the respiratory system and they are not suitable for a wide range of functional substances such as nutrients, cosmetics, and essential oils.

Other vaporization devices known in the art comprise nebulizers and diffusers. Those devices are designed for the diffusion of aromatic or therapeutic substances (such as essential oils) into the air or for drug nebulization. Although they are widely used for therapeutic purposes, nebulizers are mainly intended for local treatments (for example, for cold or for respiratory infections) and are not suitable for the administration of a wide range of active ingredients and functional substances.

Moreover, administration forms such as capsules, tablets, sprays or mouthwashes for oral, sublingual, topical administration or administration by inhalation of active ingredients in solid or liquid formulations are well known. However, those traditional methods have some limitations including dosage variability, difficulty in obtaining optimal bioavailability, and sometimes gastrointestinal side effects. Moreover, patient compliance might be reduced due to inconvenient or not very handy modalities.

The main object of the present invention is thus to provide a device that uses a vaporization technology for the administration by inhalation of a wide range of liquid formulations containing active functional substances that can be therapeutic, nutritional, cosmetic and intended for wellness, so as to overcome the drawbacks mentioned referring to the prior art.

Another object of the present invention is to provide a vaporization device for the above-mentioned use to administer the active functional substances in a more efficient, in particular in terms of bioavailability and/or absorption of the active substances, precise and rapid manner.

### Summary of the invention

The above-mentioned objects are achieved by a vaporization device suitable for the administration by inhalation of a liquid comprising at least one therapeutic, nutraceutical, food-supplement, phytotherapeutic, aromatherapeutic, cosmetic or general-wellness active ingredient and/or suitable for the administration by inhalation of a liquid classified as a medical device, the device comprising
- a tank containing said liquid,
- a vaporization unit comprising a heating element or an ultrasonic transducer to vaporize said liquid in fluid communication with said tank, said heating element being selected from a coil atomizer, a ceramic atomizer and a mesh atomizer;
- a mouthpiece connected to an end of said vaporization unit and in fluid communication with said heating element or said ultrasonic transducer,
- power-supply means in electrical communication with said heating element or said ultrasonic transducer,
- at least one opening in fluid communication with said heating element or said ultrasonic transducer to send an air flow from the outside to said heating element or said ultrasonic transducer,
- a command-and-control unit in electrical communication with said power-supply means and configured to control the temperature of said heating element or the power of said ultrasonic transducer.

The above-mentioned objects are also achieved by a kit of parts comprising:
- a vaporization device as defined above, and
- a liquid comprising at least one therapeutic, nutraceutical, food-supplement, phytotherapeutic, aromatherapeutic, cosmetic or general-wellness active ingredient or a liquid classified as a medical device.

The liquid may be contained in a suitable refill cartridge or package and is used to fill the above-mentioned device for the use according to the present invention.

Further features and advantages of the present invention will become clearer from the detailed description and from an exemplifying embodiment which are provided below by way of non-limiting illustration.

### Detailed description

In the device according to the invention, the tank contains the liquid to be vaporized. It may be integrated in the device, for example made as a single piece with the vaporization unit or it may be removable to facilitate filling or replacement. The tank may be made of different materials, such as glass, metal or plastic, depending on the chemical characteristics of the liquid and on the usage needs. The tank may be provided with filling systems that are easy to use, for example, a filling opening or a cartridge refilling system that facilitates filling with the liquid.

In the device according to the invention, the vaporization unit comprises a heating element (atomizer) or an ultrasonic transducer that constitutes the device's core in which vaporization takes place. The heating element is designed to vaporize the liquid in a controlled and safe manner, ensuring uniform vaporization without the risk of overheating or degradation of the active substances contained in the liquid. In this respect, the heating element is selected from a coil atomizer, a ceramic atomizer and a mesh atomizer.

Coil atomizers use a coil resistor to heat the liquid. The coils may be made of different materials, such as kanthal (an iron alloy composed of iron, chromium, and aluminium), nickel-chromium or stainless steel and may have different configurations (single coil, double coil, etc.) to modulate vapor production. The coil is wound around an absorbing material, such as cotton or porous ceramic, which becomes impregnated with the liquid and transports it to the coil for vaporization. The choice of the material influences electrical resistance, chemical reactivity, and coil life and allows to obtain constant and controlled vapor production.

The ceramic atomizer uses a heating element made of ceramic, an inorganic non-metal material, which provides a longer life and a more uniform heating compared to the traditional coils. Ceramic has a high chemical inertia and a good high-temperature resistance, which makes it suitable for the vaporization of different substances. Ceramic can be used both for the heating element and for the absorbing material.

The mesh atomizer uses a metal mesh as a heating element, said mesh providing a larger surface for the contact with the liquid and a more abundant vapor production. The mesh may be made of different materials, such as stainless steel or titanium, and its structure may vary in terms of hole size and density. The larger contact surface provides a faster and more uniform vaporization, thereby producing a greater amount of vapor.

The device according to the invention may include, as an alternative to a heating element as indicated above, a transducer using ultrasound technology for liquid vaporization. This system uses high-frequency sound waves to generate vibrations that act directly on the liquid, vaporizing it without the need of high heating. Ultrasound technology is particularly advantageous for the vaporization of thermolabile liquids, namely heat-sensitive substances that might degrade with the traditional heating methods. Thus, the ultrasonic transducer, which is integrated in the vaporization unit, allows to vaporize the liquid at lower temperatures, is precise and uniform and minimizes the risk of damaging the active ingredients contained in the liquid.

In the device according to the invention, the mouthpiece is the component through which the user inhales the vapor generated by the vaporization unit. The mouthpieces may have different shapes and sizes, and may be made of different materials, such as plastic or metal. The shape and the material of the mouthpiece influence user comfort and the temperature of the inhaled vapor.

In the device according to the invention, the power-supply means in electrical communication with the heating element or the ultrasonic transducer provide the energy required for device operation. Said power-supply means may consist of, for example, a rechargeable battery, typically a lithium-ion battery, or a connection to an external power-supply source, such as a USB port. Lithium-ion batteries are preferable since they provide high capacity and long duration, and also support high-power devices. The batteries may be integrated in the device or may be removable, to allow the replacement or the use of replacement batteries.

The external power supply of the device according to the invention may be made through means for connection to an external power supply, such as an AC/DC adapter or a USB port. Said connection means may be located, for example, in a base of the device and are in electrical communication with the heating element, for example through a designated electronic circuit. This type of power supply is more widespread in table devices or in high-power devices.

In the device according to the invention, the command-and-control unit comprises an electronic circuit for the electrical communication with the power-supply means and the heating element or the ultrasonic transducer and a microprocessor to actuate/stop and control the functions of the device, to process the data coming from the sensors, such as flow sensors and/or temperature sensors) and to manage the user interface, such as, for example, a display.

In accordance with an aspect of the present invention, the command-and-control unit is configured to control the temperature of said heating element or the power of the ultrasonic transducer, so as to maintain a preset temperature or power or a temperature or power within a predefined range without damaging the substances contained in the liquid.

The control of the temperature of the heating element may be carried out by configuring the command-and-control unit so as to detect the power, measured in Watt, delivered to the heating element through the connection electronic circuit, because, when the power is increased, the coil temperature and thus the amount of vapor produced increases. The microprocessor may thus adjust the power delivered.

Alternatively, the control of the temperature of the heating element may be carried out by configuring the command-and-control unit so as to detect the voltage, measured in Volt, applied to the heating element through the connection electronic circuit; in fact, the voltage influences the current passing through the coil and thus the temperature reached. In this case, the command-and-control unit adjusts the applied voltage to maintain a constant temperature, thereby ensuring a precise vaporization.

Thus, through the detection of the applied voltage or of the delivered power, the command-and-control unit may act on a power or voltage regulator of the device so as to adjust the power or the voltage and maintain a preset temperature or a temperature a predefined range.

In a further alternative, the control of the temperature of the heating element may be carried out by configuring the command-and-control unit so as to detect the coil electrical resistance, measured in Ohm (Ω). Since the resistance of the coil (or of the heating element) varies depending on the temperature, the heating-element temperature can be estimated by measuring the resistance. In this case, the command-and-control unit adjusts the voltage applied or the power delivered to the heating element depending on the detection of the heating-element resistance so as to maintain the temperature within the desired limits.

In the case the device according to the invention uses an ultrasonic transducer for liquid vaporization, a control of the flow of ultrasound vibrations is carried out with a system (for example, a sensor) that detects the intensity of the vibrations generated by the ultrasonic transducer, thereby adjusting the power delivered. The command-and-control unit monitors and adjusts the ultrasound vibrations so as to ensure that the liquid is vaporized without overheating and without damaging the heat-sensitive active ingredients. This system allows to vaporize substances that could not be otherwise treated with the traditional heating, thereby preserving the integrity of the most delicate substances.

Advantageously, thanks to the control of the heating-element temperature or of the ultrasound vibrations in the case of using an ultrasonic transducer, it is possible to adjust with precision the vaporization temperature, thereby optimizing active-ingredient absorption and preserving the chemical-physical characteristics of delicate substances (for example, vitamins, essential oils, natural extracts), which are susceptible to thermal degradation. Moreover, this system reduces the risk of overheating the vapor and thus of formation of toxic substances, thereby ensuring a safe and efficient experience for the user.

The device according to the invention further comprises at least one opening in fluid communication with the heating element or the ultrasonic transducer to send an air flow from the outside to said heating element or said ultrasonic transducer. Advantageously, the amount of air entering the vaporization unit (atomizer) may be adjusted through suitable adjustment means, for example, through an adjustment ring nut that adjusts the available orifice of the at least one opening for the entry of air.

In this way, advantageously, the user can adjust the amount of air entering the atomizer, thereby influencing the density and the temperature of the vapor produced.

The device according to the invention may also comprise one or more sensors and/or buttons in communication with the command-and-control unit to activate and/or adjust device operation. For example, the device may comprise an on/off button to activate/deactivate the heating-element heating or the ultrasonic-element vibration and/or an activation sensor detecting the user's action, such as an air flow generated by the inhalation from the mouthpiece or the pressure of an on/off button, thereby activating the electronic circuit and the command-and-control unit to start vaporization.

The device according to the invention may further comprise:
- a protection and/or safety circuit in electrical communication with the command-and-control unit to prevent short circuits, overloads, overheating, and other anomalies,
- a voltage and/or power regulator in communication with the command-and-control unit to adjust the energy flow from the power-supply means (for example, battery) to the atomizer, thereby allowing to set the power delivered or the vaporization temperature,
- a display for displaying information, in particular for displaying operation parameters of the device.

It was surprisingly found that vape-type devices, when used for the use according to the present invention, allow to efficiently and safely vaporize a liquid containing at least one therapeutic, nutraceutical, food-supplement, phytotherapeutic, aromatherapeutic, cosmetic or general-wellness active ingredient or a liquid classified as a medical device thanks to the use of a heating element selected from a coil atomizer, a ceramic atomizer, a mesh atomizer or an ultrasonic atomizer.

In fact, the above-mentioned atomizers allow a uniform and controlled heating of the liquid, thereby avoiding overheating and combustion.

Moreover, the safety and the quality of the vapor produced can be further improved using biocompatible materials to make the device for the use according to the invention, in particular, for the vaporization unit and/or the tank, such as medical-grade stainless steel, borosilicate glass, and food-grade plastics.

Moreover, the safety of the device is ensured by the protection circuits, by the temperature-control systems, and also by the possible use of biocompatible materials. In fact, the protection circuits prevent short circuits, overloads, and overheating, the temperature-control systems avoid liquid combustion and toxic-substance formation and the biocompatible materials ensure that the device does not release harmful substances during use.

The present invention is different from the prior art because of its innovative approach that uses vape-type devices to vaporize and administer beneficial substances (including drugs, nutraceuticals, cosmetics, and essential oils), thereby overcoming the limitations of the existing technologies and paving the way for a versatile and safe use for a wide range of therapeutic and wellness applications.

Moreover, the present invention also allows a controlled and rapid administration with an improved bioavailability, thanks to the vaporization that preserved the integrity of the active substances. Furthermore, the possibility of choosing from different vaporization modalities, including vaporization through ultrasound and vaporization by heating, ensures maximum flexibility in the use of the device for a broad range of therapeutic active ingredients, nutraceuticals, food supplements, medical devices, phytotherapeutics, aromatherapeutics, functional active substances, cosmetics.

Moreover, the present invention allows to improve patient compliance, since the vaporization devices are generally easy to use, can be used in any place and at any time, and are also suitable for patients having swallowing difficulties.

In the device according to the present invention, the liquid to be vaporized contains at least one therapeutic, nutraceutical, food-supplement, phytotherapeutic, aromatherapeutic, cosmetic or general-wellness active ingredient or a liquid to be vaporized is a liquid classified as a medical device. In any case, the liquid to be vaporized is nicotine free, whereas, by contrast, nicotine is contained in the liquids that are traditionally used in vape-type devices.

Non-limiting examples of liquids containing one or more active ingredients usable in the device according to the invention comprise:
- therapeutic liquids, namely, solutions containing pharmaceutical active ingredients for the treatment of different pathologies,
- liquids classified as medical devices, such as wound-cleaning solutions, ophthalmic solutions or solutions for inhalation,
- drugs containing pharmaceutical active ingredients in a liquid or solubilized form, intended for systemic or local administration,
- liquids containing substances promoting the physical and mental wellness, such as aromas, essential oils, adaptogens, and nootropics.

Therapeutic liquids and drugs comprise a wide range of liquid solutions containing pharmaceutical active ingredients, intended for the treatment of different pathologies. Their formulation is specifically studied to ensure the stability and effectiveness of the active ingredient during vaporization and administration by inhalation.

The main categories of therapeutic liquids and drugs usable in the device according to the invention comprise:
- airway drugs comprising active ingredients acting on airways for the treatment of pathologies such as asthma, bronchitis, COPD, cystic fibrosis, and other pulmonary diseases; they may include drugs with different pharmacological actions, such as bronchodilators, corticosteroids, anti-inflammatory drugs, antibiotics, antivirals, and mucolytic drugs.
- drugs for the oro-pharyngeal cavity containing active ingredients acting on the oral cavity and the pharynx for the treatment of pathologies such as sore throat, pharyngitis, tonsillitis, aphthae, gingivites, and other infections or inflammations of the oral cavity; they may include drugs with different pharmacological actions, such as antiseptics, anti-inflammatory drugs, analgesics, local anesthetics, and antimycotic drugs,
- cardiovascular drugs containing active ingredients acting on the cardiovascular system for the treatment of pathologies such as hypertension, angina pectoris, arrhythmiae, cardiac insufficiency, and other heart diseases; they may include drugs with different pharmacological actions, such as antihypertensives, antianginals, antiarrhythmic drugs, diuretics, inotropes, and antithrombotic drugs,
- neurological drugs containing active ingredients acting on the central or peripheral nervous system for the treatment of pathologies such as epilepsy, Parkinson's disease, Alzheimer's disease, multiple sclerosis, chronic pain, and other neurological diseases; they may include drugs with different pharmacological actions, such as antiepileptic drugs, antiparkinson drugs, antidepressants, anxiolytics, antipsychotics, analgesics, and neuroprotective drugs,
- anti-infective drugs containing active ingredients acting against different types of pathogenic microorganisms, such as bacteria, viruses, fungi, and parasites; they may include drugs with different pharmacological actions, such as antibiotics, antivirals, antifungals, and antiparasitics,
- other drugs containing active ingredients acting on different systems and apparatuses of the human body, for the treatment of a wide range of pathologies; they may include drugs with different pharmacological actions, such as anesthetics, anti-inflammatory drugs, antiallergics, immunosuppressive drugs, hormones, antitumor drugs and drugs for the treatment of metabolic, endocrine and gastrointestinal diseases.

The medical devices include a wide range of liquid devices or devices in solution, specifically formulated to be vaporized and administered by inhalation, with the purpose of acting locally on the oral cavity and on the upper airways. Those medical devices are designed to provide a wide range of therapeutic actions, preventive actions and action supporting the physiological functions, with particular focus on the protection, the alleviation, and the treatment of the diseases of the oral cavity and of the upper airways.

The main categories of liquids classified as medical devices usable in the device according to the invention comprise:
- Devices for oral-cavity care, such as:
   a) Solutions based on chlorhexidine, cetylpyridinium chloride or other antiseptics for the prevention and the treatment of oral-cavity infections, such as gingivites, stomatitises, aphthae, and halitosis,
   b) Solutions containing hyaluronic acid, aloe vera or other -hydrating and lenitive agents for the treatment of aphthae, oral mucosa lesions, dry mouth, and irritations caused by dentures or orthodontic braces,
   c) Solutions containing fluorine or other remineralizing agents for caries prevention and tooth-enamel protection,
   d) Solutions containing proteolytic enzymes or other agents for dental-plaque removal and oral-hygiene maintenance,
   e) Solutions containing local anesthetics, such as lidocaine or benzocaine, for the treatment of oral pain and gums.
- Devices for upper-airway care, such as:
   a) Isotonic or hypertonic saline solutions for airway hydration and cleaning, which are useful in the case of dry nose, congestion, rhinitis, sinusitis and other respiratory diseases,
   b) Solutions containing mucoactive or mucolytic active ingredients to fluidify mucus and facilitate the expectoration thereof, which are useful in the case of coughing, bronchitis, cold, and other respiratory pathologies with mucus production,
   c) Solutions containing antiseptics or disinfectants for the treatment of airway infections, such as pharyngitides, laryngitides, and tracheitis,
   d) Solutions containing anti-inflammatory or antiallergic active ingredients for the treatment of allergic rhinitides, sinusitis, and other airway inflammations,
   e) Solutions containing local anesthetics, such as lidocaine or benzocaine, for the treatment of pain and irritation of the throat and upper airways.
- Devices based on natural substances, such as:
   a) Phytotherapeutic devices: Solutions containing extracts of medicinal plants, such as propolis, echinacea, chamomile, mallow, eucalyptus, mint, tea tree oil, calendula, etc., with antiseptic, anti-inflammatory, lenitive, balsamic, expectorant, antioxidant and immunostimulating properties,
   b) Devices based on other natural ingredients: Solutions containing other ingredients of natural origin, such as honey, propolis, aloe vera, hyaluronic acid of vegetable origin, essential oils, etc., with hydrating, lenitive, protective, antioxidant and antimicrobial properties. Those devices may be used for the prevention and the treatment of several diseases of the oral cavity and of the upper airways, such as sore throat, coughing, cold, sinusitis, gingivites, aphthae, irritations, dry mouth, and halitosis. They may also be used to promote tissue regeneration, to protect the mucosae from external agents, to reinforce the immune system, and to promote the general wellness of the oral cavity and of the airways.

The liquids containing substances for the physical wellness comprise a wide range of liquids containing substances promoting the physical and mental wellness, specifically formulated to be vaporized and administered by inhalation. Although those substances do not have a specific therapeutic action, they may contribute to the improvement of the general health status, by promoting relaxation, improving the mood, increasing concentration or supporting cognitive functions.

The main categories of wellness substances usable in the device according to the invention comprise:
- Aromas such as natural or synthetic aromas, selected for their sensory properties and for their capability of influencing the mood and emotions; by way of a non-limiting example, they may include aromas of lavender, chamomile, vanilla, citruses, mint, etc.
- Essential oils such as concentrated extracts of aromatic plants, obtained by steam distillation or other extraction methods; the essential oils contain different bioactive molecules, such as terpenes, alcohols, aldehydes, and ketones, which may have beneficial effects on physical and mental health. By way of a non-limiting example, they may include essential oils of lavender, chamomile, melissa, mint, eucalyptus, tea tree oil, etc,
- Adaptogens, namely, natural substances that help the organism adapt to stress and maintain homeostasis; by way of a non-limiting example, they may include extracts of ginseng, ashwagandha, rhodiola, eleutherococcus, etc,
- nootropics, namely, substances that improve cognitive functions, such as memory, concentration, learning and creativity; by way of a non-limiting example, they may include choline, caffeine, L-theanine, racetam, etc,
- other substances such as, for example, melatonin for sleep-wakefulness regulation, 5-HTP (5-hydroxytryptophan) for mood improvement, GABA ((gamma-aminobutyric acid) for a relaxing effect, etc.

Wellness substances may be used for different purposes, for example:
- to promote relaxation and reducing stress: Aromas or essential oils such as lavender, chamomile and melissa can have a soothing and relaxing effect, thereby helping reducing stress and anxiety,
- to improve mood: Aromas and essential oils such as citruses, mint, and ylang ylang can have a stimulating and euphorizing effect, thereby helping improving mood and fighting depression,
- to increase concentration and productivity: Substances such as caffeine, L-theanine, and nootropics can improve concentration, memory, and cognitive functions, thereby promoting productivity and learning,
- to support sleep: Substances such as melatonin and valerian can promote relaxation and sleep, thereby helping fighting insomnia and improving night-rest quality,
- to promote general wellness: The use of wellness substances can contribute to improving life quality, by increasing vitality, energy and general-wellness sensation.

Non-limiting examples of liquids containing one or more nutraceutical active ingredients usable in the device according to the invention comprise:
- nutraceuticals, namely, products in a liquid or solubilized form having active ingredients with nutritional and therapeutic properties, such as vegetable extracts, antioxidants, and probiotics, prebiotics, and other bioactive compounds,
- supplements, namely liquid or solubilized formulations containing vitamins, minerals, amino acids, essential fatty acids, and other nutritious substances.

The main categories of nutraceuticals usable in the device according to the invention comprise:
- Vegetable extracts: extracts of medicinal plants, such as turmeric, ginseng, ginkgo biloba, echinacea, etc., containing active ingredients with antioxidant, anti-inflammatory, immunomodulatory and adaptogen properties and other activities that are beneficial to health.
- antioxidants: substances protecting cells from oxidative damages caused by free radicals, such as vitamin C, vitamin E, coenzyme Q10, flavonoids, carotenoids, and polyphenols,
- probiotics: living microorganisms which, when administered in suitable amounts, benefit host health, such as Lactobacillus and Bifidobacterium, which promote the balance of intestinal bacterial flora and digestive-system health.
- Prebiotics: non-digestible dietary fibers promoting the growth and the activity of probiotics, such as inulin, fructooligosaccharides, and galactooligosaccharides,
- other bioactive compounds such as, for example, enzymes, coenzymes, polyunsaturated fatty acids, phospholipids, vegetable sterols, etc., with different activities beneficial for the health.

Supplements comprise a wide range of liquid formulations containing essential nutrients, such as vitamins, minerals, amino acids, essential fatty acids, and other nutritious substances. Said liquid formulations are specifically formulated to be vaporized and administered by inhalation into the oral or nasal cavity, thereby ensuring - in accordance with the present invention - efficient nutrient transmission and optimal bioavailability.

The main categories of nutrients usable in the device according to the invention comprise:
- Liposoluble (A, D, E, K) and hydrosoluble (C and B-complex) vitamins which are essential for different physiological functions, such as growth, energetic metabolism, immune function, and cell protection,
- Macroelements (calcium, magnesium, sodium, potassium, etc.) and microelements (iron, zinc, selenium, etc.) which are essential for bone structure, muscle function, water balance, enzymatic function, and other biological processes,
- essential and non-essential amino acids, the fundamental constituents of proteins, which are necessary for growth, tissue repair, production of enzymes, hormones and antibodies.
- Essential fatty acids, such as omega-3 and omega-6 fatty acids, which are important for cardiovascular health, cerebral function, inflammatory response, and other physiological processes,
- other nutritious substances, such as, for example, antioxidants, such as vitamin C, vitamin E, coenzyme Q10 and flavonoids, which protect cells from oxidative damages; probiotics, living microorganisms promoting the balance of oral and intestinal bacterial flora and digestive-system health; prebiotics, non-digestible dietary fibers promoting the growth and the activity of probiotics; phytonutrients, bioactive compounds present in plants, with antioxidant, anti-inflammatory and immunomodulatory properties.

The formulation of the liquids used with the device according to the invention is suitably carried out by the person skilled in the art so as to ensure nutrient bioavailability and absorption by inhalation. In this respect, the choice of the excipients, such as solvents, surfactants, preservatives, and flavoring agents, may be made by the person skilled in the art based on his/her general knowledge, so as to obtain a stable and biocompatible formulation suitable for vaporization, considering the main factors to be considered in the formulation, such as solubility of the nutrients and their chemical stability in the carrier liquid, liquid viscosity that must be suitable for effective vaporization and good penetration into the airways, surface tension of the liquid, pH, osmolarity, and sterility.

Hereinafter, by way of example, there is the description of some examples of devices that can be used for the use according to the invention for the vaporization and administration of a liquid containing at least one therapeutic, nutraceutical, food-supplement, phytotherapeutic, aromatherapeutic, cosmetic active ingredient or of a liquid classified as a medical device.

The above-mentioned examples are provided by way of a non-limiting example with reference to the accompanying figures in which:

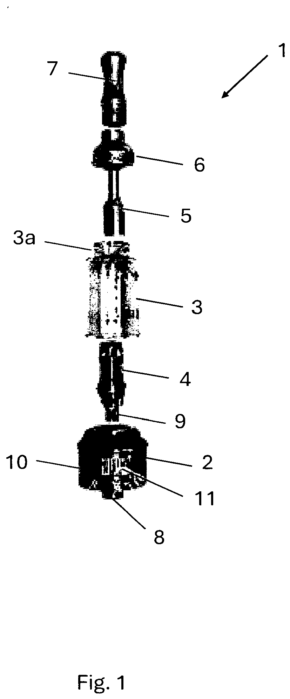

- Figure 1 shows an exploded view of a device suitable for the vaporization and administration, in accordance with the present invention, of a liquid containing at least one therapeutic, nutraceutical, food-supplement, phytotherapeutic, aromatherapeutic, cosmetic active ingredient or of a liquid classified as a medical device,

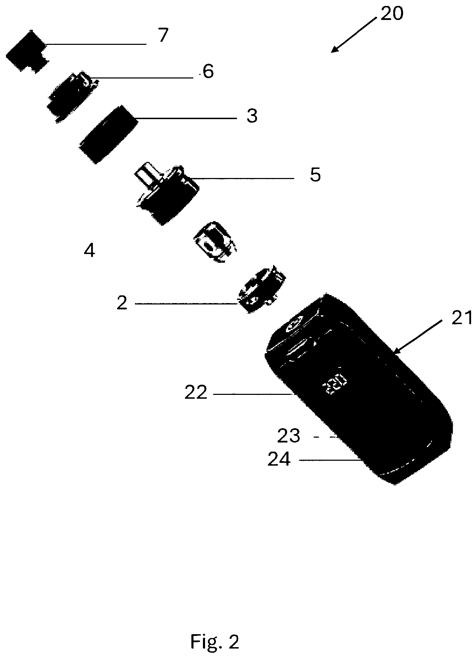

- Figure 2 shows an exploded view of another device suitable for the vaporization and administration, in accordance with the present invention, of a liquid containing at least one therapeutic, nutraceutical, food-supplement, phytotherapeutic, aromatherapeutic, cosmetic active ingredient or of a liquid classified as a medical device.

With reference to Figure 1, a device suitable for the vaporization and administration, in accordance with the present invention, of a liquid containing at least one therapeutic, nutraceutical, food-supplement, phytotherapeutic, aromatherapeutic, cosmetic active ingredient or of a liquid classified as a medical device is referred to as a whole with reference number 1.

The device 1 comprises a base 2, a tank 3 intended to contain a liquid containing at least one therapeutic, nutraceutical, food-supplement, phytotherapeutic, aromatherapeutic, cosmetic active ingredient or of a liquid classified as a medical device, a heating element 4 consisting of a coil atomizer, a chimney 5, a cap 6, and a mouthpiece 7.

The base 2 has at one end a port 8 for the connection to a power-supply source (for example, a removable battery, not shown) and contains a command-and-control unit and an electronic circuit (both not shown) in electrical communication with the port 6. The heating element 4 has a connector 9 for the mechanical connection to the base 2 and the electrical connection to the port 7 of the base 2. In the assembled device 1, the heating element 4 is covered by the chimney 5, thereby defining a vaporization chamber or area between the heating element 4 and the internal part of the chimney 5.

The tank 3 has a substantially cylindrical tubular shape tapering at the top in a tapered end region 3a provided with an external thread. The tank 3 may be suitably made of glass and may be sealed to the base 2 at an end thereof that is opposite the end having the port 6, and it is closed at the top by the cap 5, which is internally provided with a thread, through screwing of the cap 5 on the tapered region 3a of the tank, thereby making a threaded coupling between the external thread of the tapered region 3a and the internal thread of the cap 5.

More in detail, the chimney 5 has a tubular shape with a lower portion having a larger diameter that covers the heating element and an elongated upper portion that comes out at the top from the upper end of the tank 3 passing through internally the cap 6 and part of the mouthpiece 7.

The cap 5 is tubular and substantially bell-shaped with a lower portion having a larger diameter that has internally a thread for screwing/unscrewing on the tapered upper portion 3a of the tank 3 and an upper portion having a smaller diameter on which the mouthpiece 7 is connected. The cap 6 is provided with at least one closeable opening (not shown) for filling or refilling the liquid in the tank 3.

The vaporization unit comprising the heating element is suitably in fluid communication with the tank 3 and with at least one internal channel of the base 2 ending in an opening 10 for the entry of air from the outside to the heating element 4. The base is also provided with an adjustment ring nut 11 from the orifice of the opening 10 so as to adjust the air flow to the heating element 4.

Figure 2 shows another device suitable for the vaporization and administration, in accordance with the present invention, of a liquid containing at least one therapeutic, nutraceutical, food-supplement, phytotherapeutic, aromatherapeutic, cosmetic active ingredient or of a liquid classified as a medical device, wherein said device is referred to as a whole with reference number 20.

The features of the device 20 that are structurally and/or functionally equivalent to corresponding features of the device 1 described above will be assigned the same reference numbers of the latter and, for the sake of brevity, will not be further described.

Compared to the device 1 described above, the device 20 is characterized in that it has a battery 21 connectable in a removable manner to the base 2 and provided with a turning-on button 22, buttons 23 for setting/operating the device and a USB port 24.

In the light of the above, the device according to the invention provides several advantages that make it particularly suitable for the vaporization and the administration of a wide range of active liquids in the field of health and wellness.

Indeed, the vaporization carried out with the device according to the invention allows to administer the active ingredients bypassing the gastrointestinal tract and the hepatic-first-pass effect, which can degrade or inactivate many substances. This results in a more rapid and complete absorption of the active ingredients, with greater bioavailability and a more rapid action.

Moreover, the vaporization with the device according to the invention reduces the risk of gastrointestinal side effects, such as nausea, vomiting, and diarrhea, because the active ingredients do not come in contact with the digestive tract. Vaporization can thus be a good alternative for patients that do not tolerate oral administration of therapeutic, nutraceutical, food-supplement, phytotherapeutic, aromatherapeutic active ingredients and of liquids classified as a medical device.

The vaporization device according to the invention also has the advantage that it is easy to use and that can be used in any place and at any time, without the need for preparation. This aspect can significantly improve patient compliance, especially in the case of long-term administrations or of patients having swallowing difficulties or if other medical devices are used, thereby promoting compliance to the therapy.

Moreover, the device used according to the invention may be used to administer a wide range of substances, with different chemical-physical characteristics and different therapeutic or wellness purposes. The possibility to formulate liquids with different concentrations, viscosities and surface tensions allows to adjust vaporization to the specific needs of each substance and of each functionality. Moreover, vaporization may be used to administer both lipophilic and hydrophilic substances, thereby further expanding the possibilities of application.

Finally, vaporization allows to administer active ingredients in a way that targets the respiratory system, directly reaching the involved areas. This aspect is particularly advantageous in the treatment of respiratory diseases, such as asthma or brochitis, where local administration of the drug may increase effectiveness and reduce systemic side effects.

Based on the above, the device according to the invention advantageously finds different applications for the vaporization and administration of active liquids in different fields, including human medicine, aromatherapy, cosmetic, dentistry, wellness and fitness, and prevention.

A person skilled in the art may make numerous modifications and changes to the device according to the invention, which, however, are all within the scope of protection of the following claims.

## Claims

1. A device (1;20) for the vaporization and the administration by inhalation of a liquid comprising:
- a tank (3) containing a liquid to be vaporized, said liquid comprising at least one therapeutic, nutraceutical, food-supplement, phytotherapeutic, aromatherapeutic, cosmetic or general-wellness active ingredient or said liquid being a liquid classified as a medical device,
- a vaporization unit comprising a heating element (4) or an ultrasonic transducer to vaporize said liquid in fluid communication with said tank (3), said heating element being selected from a coil atomizer, a ceramic atomizer and a mesh atomizer;
- a mouthpiece (7) connected to an end of said vaporization unit and in fluid communication with said heating element (4) or said ultrasonic transducer,
- power-supply means in electrical communication with said heating element (4) or said ultrasonic transducer,
- at least one opening (10) in fluid communication with said heating element (4) or said ultrasonic transducer to send an air flow from the outside to said heating element (4) or said ultrasonic transducer,
- a command-and-control unit in electrical communication with said power-supply means and configured to control the temperature of said heating element (4) or the power of said ultrasonic transducer.

2. The device (1;20) according to claim 1, wherein said power-supply means comprise a battery (21), preferably a rechargeable battery.

3. The device (1;20) according to claim 1 or 2, wherein said command-and-control unit is configured to detect the power delivered or the voltage applied to said heating element (4) or it is configured to detect the resistance of said heating element and to adjust said power or said voltage so as to maintain a preset temperature or a temperature a predefined range.

4. The device (1;20) according to claim 1 or 2, wherein said command-and-control unit is configured to detect and adjust the power delivered by said ultrasonic transducer.

5. The device (1;20) according to any one of the preceding claims, further comprising adjustment means, preferably an adjustment ring nut, to adjust the available orifice of said at least one opening (10) for the entry of air toward said heating element (4) or said ultrasonic transducer.

6. The device (1;20) according to any one of the preceding claims, further comprising a protection and/or safety circuit in electrical communication with the command-and-control unit.

7. The device (1;20) according to any one of the preceding claims, further comprising a voltage and/or power regulator in communication with the command-and-control unit to adjust the energy flow from said power-supply means to said heating element (4) or said ultrasonic transducer.

8. The device (1;20) according to any one of the preceding claims, further comprising a display for displaying information.

9. The device (1;20) according to any one of the preceding claims, **characterized in that** it is made of biocompatible materials preferably selected from medical-grade stainless steel, borosilicate glass, food-grade plastics, and combinations thereof.

10. The device (1;20) according to any one of the preceding claims, wherein said liquid to be vaporized is selected from:
- a therapeutic liquid containing at least one pharmaceutical active ingredient for the treatment of at least one pathology,
- a liquid classified as a medical device, preferably a wound-cleaning solution, an ophthalmic solution or a solution for inhalation,
- a drug in a liquid or solubilized form containing at least one pharmaceutical active ingredient intended for systemic or local administration,
- a liquid containing at least one substance promoting the physical and mental wellness, wherein said at least one substance is preferably selected from aromas, essential oils, adaptogens, and nootropics.

11. A kit of parts comprising:
1) a device (1;20) for the vaporization and the administration by inhalation of a liquid comprising:
- a tank (3) intended to contain a liquid to be vaporized,
- a vaporization unit comprising a heating element (4) or an ultrasonic transducer to vaporize said liquid in fluid communication with said tank (3), said heating element being selected from a coil atomizer, a ceramic atomizer and a mesh atomizer;
- a mouthpiece (7) connected to an end of said vaporization unit and in fluid communication with said heating element (4) or said ultrasonic transducer,
- power-supply means in electrical communication with said heating element (4) or said ultrasonic transducer,
- at least one opening (10) in fluid communication with said heating element (4) or said ultrasonic transducer to send an air flow from the outside to said heating element (4) or said ultrasonic transducer,
- a command-and-control unit in electrical communication with said power-supply means and configured to control the temperature of said heating element (4) or the power of said ultrasonic transducer, and
2) a liquid comprising at least one therapeutic, nutraceutical, food-supplement, phytotherapeutic, aromatherapeutic, cosmetic or general-wellness active ingredient or a liquid classified as a medical device.
